# EUROPÄISCHE PATENTSCHRIFT

(11) **EP 3 577 098 B1**
(45) Veröffentlichungstag und Bekanntmachung des Hinweises auf die Patenterteilung: **28.04.2021**
(21) Anmeldenummer: 18714149.4
(22) Anmeldetag: 12.03.2018
(51) Int. Cl.: C07C 29/151

(54) **MISCHUNG ZUR VERWENDUNG ALS FLÜSSIGES SORPTIONSMITTEL BEI DER METHANOL-SYNTHESE UND VERFAHREN ZUR METHANOL-SYNTHESE UNTER VERWENDUNG DER MISCHUNG**
MIXTURE FOR USE AS A LIQUID SORPTION AGENT IN METHANOL SYNTHESIS AND METHANOL SYNTHESIS PROCESS USING SAID MIXTURE
MÉLANGE DESTINÉ À ÊTRE UTILISÉ EN TANT QU'AGENT DE SORPTION LIQUIDE LORS DE LA SYNTHÈSE DE MÉTHANOL, ET PROCÉDÉ DE SYNTHÈSE DE MÉTHANOL AU MOYEN DE CE MÉLANGE

(30) Priorität: 14.03.2017 DE 102017204226
(43) Veröffentlichungstag der Anmeldung: 11.12.2019
(73) Patentinhaber: Siemens Energy Global GmbH & Co. KG, 81739 München (DE)
(72) Erfinder: ALBERT, Jakob, 91094 Erlangen (DE); BALDAUF,Manfred, 91056 Erlangen (DE); REICHERT, Jenny, 97523 Schwanfeld (DE); MELTZER, Katharina, 91052 Erlangen (DE); TREMEL, Alexander, 91096 Möhrendorf (DE); WASSERSCHEID, Peter, 91054 Erlangen (DE)
(86) Internationale Anmeldenummer: PCT/EP2018/055997
(87) Internationale Veröffentlichungsnummer: WO 2018/166933

(56) Entgegenhaltungen:
- EP-A1- 2 653 457
- WO-A1-2017/162512
- WO-A2-2010/107929
- DE-A1-102009 038 690
- DE-A1-102015 215 662
- SELVAMANI V ET AL: "Asymmetric tetraalkyl ammonium cation-based ionic liquid as an electrolyte for lithium-ion battery applications", JOURNAL OF SOLID STATE ELECTROCHEMISTRY, SPRINGER, BERLIN, DE, Bd. 20, Nr. 8, 21. Mai 2016 (2016-05-21), Seiten 2283-2293, XP036004900, ISSN: 1432-8488, DOI: 10.1007/S10008-016-3248-X [gefunden am 2016-05-21]
- OSAMU SHIMAMURA, NOBUKO YOSHIMOTO, MAMI MATSUMOTO, MINATO EGASHIA, MASAYUKI MORITA: "Electrochemical co-deposition of magnesium with lithium from quaternary ammonium-based ionic liquid", JOURNAL OF POWER SOURCES, Bd. 196, 21. September 2010 (2010-09-21), Seiten 1586-1588, XP002781641, DOI: https://doi.org/10.1016/j.jpowsour.2010.08 .060
- HENG ZHANG ET AL: "Recent progresses on electrolytes of fluorosulfonimide anions for improving the performances of rechargeable Li and Li-ion battery", JOURNAL OF FLUORINE CHEMISTRY, Bd. 174, 1. Juni 2015 (2015-06-01), Seiten 49-61, XP055271810, NL ISSN: 0022-1139, DOI: 10.1016/j.jfluchem.2014.07.028
- UEDA MIKITO ET AL: "Electrorefining reaction of sodium in sodium-bis(trifluoremethylsulfonyl)imide and tetraethylammonium-bis(trifluoremethylsulf onyl)imide mixture ionic liquid", ELECTROCHIMICA ACTA, ELSEVIER SCIENCE PUBLISHERS, BARKING, GB, Bd. 100, 30. Oktober 2012 (2012-10-30), Seiten 265-268, XP028544116, ISSN: 0013-4686, DOI: 10.1016/J.ELECTACTA.2012.10.097
- MARZIEH BARGHAMADI ET AL: "Lithium-sulfur batteries-the solution is in the electrolyte, but is the electrolyte a solution?", ENERGY & ENVIRONMENTAL SCIENCE, Bd. 7, Nr. 12, 1. Januar 2014 (2014-01-01) , Seiten 3902-3920, XP055448469, Cambridge ISSN: 1754-5692, DOI: 10.1039/C4EE02192D
- A. ARCE ET AL.: "Mutually immicible ionic liquids", CHEM. COMMUN., 16. Mai 2006 (2006-05-16), Seiten 2548-2550, XP002783992,
- KAZUKI YOSHII ET AL: "Physicochemical Properties of Tri -n -butylalkylphosphonium Cation-Based Room-Temperature Ionic Liquids", JOURNAL OF PHYSICAL CHEMISTRY PART B: CONDENSED MATTER, MATERIALS, SURFACES, INTERFACES & BIOPHYSICAL, Bd. 117, Nr. 48, 5. Dezember 2013 (2013-12-05), Seiten 15051-15059, XP055285398, US ISSN: 1520-6106, DOI: 10.1021/jp406791a

## Beschreibung

Die vorliegende Erfindung betrifft eine Mischung zur Verwendung als flüssiges Sorptionsmittel bei der Methanol-Synthese sowie ein Verfahren zur Durchführung einer Methanol-Synthese unter Verwendung der Mischung.

Bei vielen chemischen Synthesen treten sogenannte gleichgewichtslimitierende Reaktionen auf, d.h. die Synthese von Produkten stoppt (von außen betrachtet), wenn eine Gleichgewichtslage zwischen Edukten und Produkt(en) erreicht ist. Bei derartigen Synthesen ist es daher für einen hohen Umsetzungsgrad von Vorteil, Produkt(e) aus dem Synthese-System zu entfernen, um so die Einstellung einer Gleichgewichtslage zwischen Edukten und Produkt(en) zu vermeiden oder um von einer Gleichgewichtslage wieder zu einer Ungleichgewichtslage zu kommen, was etwa durch eine kontinuierliche oder diskontinuierliche Entfernung von Produkt(en) aus dem Reaktionssystem erreicht werden kann.

Zwei bekannte Beispiele für eine chemische Synthese unter gleichgewichtslimitierender Reaktion stellen die Erzeugung von Methanol aus Wasserstoff und Kohlenstoffmonoxid gemäß nachfolgender Reaktionsgleichung 1. oder aus Wasserstoff und Kohlenstoffdioxid gemäß nachfolgender Reaktionsgleichung 2. dar:
1. 2H₂ + CO ⇄ CH₃OH
2. 3H₂ + CO₂ ⇄ CH₃OH + H₂O

Aktuell findet die Synthese von Methanol vor allem in heterogenen, katalysierten Festbett-Reaktoren statt, wobei die Edukte bei einem einfachen Durchgang durch den Reaktor nur teilweise umgesetzt werden. Die kondensierbaren Reaktionsprodukte werden daher nach einem Durchgang abgetrennt und die nicht umgesetzten Edukte zum Reaktionseintritt rezirkuliert. Die Rezirkulation der teilweise großen Gasmengen führt zu einem hohen apparativen Aufwand.

Zur Überwindung oder doch Verringerung dieses Nachteils ist aus der DE 10 2015 202 681 A1 ein Verfahren zur Durchführung einer chemischen Synthese unter Verwendung von zwei nur teilweise ineinander löslichen Trägerphasen und einem Katalysator, der in einer der Trägerphasen dispergierter vorliegt, bekannt, bei dem die Trägerphasen in einem Reaktor vermischt werden, wenigstens ein Syntheseedukt in den Reaktor eingeleitet wird und die beiden Trägerphasen anschließend voneinander getrennt werden.

Bei der Methanol-Synthese weisen das Syntheseprodukt Methanol bzw. die Syntheseprodukte Methanol und Wasser eine Affinität zu polaren Flüssigkeiten auf, so dass sich das Syntheseprodukt bzw. die Syntheseprodukte in solchen Flüssigkeiten anreichern. Daher ist gemäß der genannten Druckschrift bei der Methanol-Synthese eine der Trägerphasen bevorzugt eine polare Flüssigkeit, die gebildet sein kann aus ionischer Flüssigkeit/ionischen Flüssigkeiten, polarem Lösungsmittel/polaren Lösungsmitteln oder auch einem oder mehreren höhermolekularen Alkoholen.

In der polaren Trägerphase reichert sich das Syntheseprodukt/reichern sich die Syntheseprodukte an, wodurch bis zum Erreichen der Sättigungsgrenze in der polaren Trägerphase die Einstellung einer stationären Gleichgewichtssituation in der anderen, weniger polaren oder unpolaren Trägerphase, die (zumindest überwiegend) die Edukte und den für die Methanol-Synthese erforderlichen Katalysator aufweist, verhindert wird.

In einem weiteren Verfahrensschritt werden die beiden Trägerphasen voneinander getrennt, indem bspw. die polare Trägerphase aus dem Reaktor ausgeleitet wird, und es werden das Syntheseprodukt/die Syntheseprodukte (Methanol bzw. Methanol und Wasser) zumindest teilweise aus der polaren Trägerphase abgetrennt. Die von den Syntheseprodukten "befreite" polare Trägerphase kann danach wieder in den Reaktor zurückgeführt werden.

Bei den als mögliche Trägerphase genannten ionischen Flüssigkeiten handelt es sich um bei niedrigen Temperaturen (<100°C) schmelzende Salze, die eine Klasse von Lösungsmitteln mit extrem geringem Dampfdruck darstellen.

Auch wenn erste Vertreter schon seit 1914 bekannt sind, werden ionische Flüssigkeiten erst seit etwa 15 Jahren intensiv als Lösungsmittel für chemische Umsetzungen untersucht. Der äußerst geringe Dampfdruck ionischer Flüssigkeiten ist für bestimmte Verfahren von großem Vorteil, da auf diese Weise eine extraktive Trennung eines Reaktionsgemisches möglich ist, ohne die Gasphase mit Lösungsmitteldämpfen zu verunreinigen. Die Rückgewinnung des Extrakts aus der ionischen Flüssigkeit ist oftmals durch einfache Destillation möglich, wobei Probleme wie eine Azeotrop-Bildung zwischen Lösungsmittel und Extrakt nicht auftreten.

Durch geeignete Wahl von Kation und Anion einer ionischen Flüssigkeit sind eine gezielte Einstellung ihrer Polarität und damit eine Abstimmung ihrer Löslichkeitseigenschaften möglich. Die Bandbreite reicht dabei von wassermischbaren ionischen Flüssigkeiten über wassernichtmischbaren ionischen Flüssigkeiten bis hin zu solchen, die selbst mit organischen Lösungsmitteln oder anderen ionischen Flüssigkeiten zwei Phasen bilden. Eine geschickte Ausnutzung der außerordentlichen Löslichkeitseigenschaften ist der Schlüssel zum erfolgreichen Einsatz von ionischen Flüssigkeiten als neuartige Lösungsmittelklasse.

Probleme bei der Verwendung von ionischen Flüssigkeiten können sich bei höheren Temperaturen ergeben, da viele der ionischen Flüssigkeiten oberhalb von 200°C nicht dauerhaft temperaturstabil sind. Auch kann die Anwesenheit von Katalysatoren, Reaktanden und Reaktionsprodukten eine signifikante Einschränkung der Kation- und Anionauswahl unter Stabilitätsgesichtspunkten zur Folge haben.

P. Wasserscheid, W. Keim: "Ionische Flüssigkeiten - neue "Lösungen" für die Übergangsmetallkatalyse", Angew. Chem., Band 112, Ausgabe 21, S. 3926-3945, 03. November 2000, beschreibt die Verwendung ionischer Flüssigkeiten als Extraktionsmedien in der Zweiphasen-Katalyse.

Auch wenn aus dem Stand der Technik ionische Flüssigkeiten grundsätzlich bereits als mögliche Trägerphase im Zusammenhang mit der Methanol-Synthese erwähnt sind (siehe WO 2010/107929 A2 bzw. DE 10 2015 215662 A1), stellt die Verwendung von ionischen Flüssigkeiten als Sorptionsmittel (Extraktionsphase) von Methanol bzw. Methanol und Wasser unter den Reaktionsbedingungen der Methanol-Gasphasensynthese unter Verwendung von Kohlenmonoxid und Wasserstoff, Kohlendioxid und Wasserstoff oder einer Mischung aus Kohlenmonoxid, Kohlendioxid und Wasserstoff ein bisher nicht gelöstes Problem dar.

Hierbei sind nämlich besonders hohe Anforderungen an die Stabilität der eingesetzten ionischen Flüssigkeit(en) bei bis zu 300°C und bis zu 300 bar Synthesegasdruck in Gegenwart eines Katalysators, wie etwa Cu-Zn-Oxid auf Aluminiumoxid, und der Reaktionsprodukte gegeben.

Auch wäre es von Vorteil, wenn die ionische Flüssigkeit bei einer Methanol-Synthese unter Verwendung von Kohlendioxid und Wasserstoff auch ein möglichst gleichgutes Sorptionsverhalten in Bezug die Syntheseprodukte Wasser und Methanol besitzen würde, um eine Akkumulation von einem der Syntheseprodukte in der Gasphase zu vermeiden.

Vor diesem Hintergrund ist es Aufgabe der vorliegenden Erfindung, neuartige Sorptionsmittel für Methanol oder Methanol und Wasser bei der Methanol-Synthese auf Basis von ionischen Flüssigkeiten sowie ein Verfahren zur Methanol-Synthese unter Verwendung derartiger Sorptionsmittel bereitzustellen.

Diese Aufgaben werden gelöst durch die Mischung gemäß Anspruch 1 und das Verfahren gemäß Anspruch 7. Vorteilhafte Weiterbildungen der Mischung und des Verfahrens sind Gegenstand der Unteransprüche.

Gemäß der vorliegenden Erfindung wird eine Mischung zur Verwendung als flüssiges Sorptionsmittel für Methanol oder Methanol und Wasser bei der Methanol-Synthese unter Verwendung von Kohlenmonoxid und Wasserstoff, Kohlendioxid und Wasserstoff oder einer Mischung aus Kohlenmonoxid, Kohlendioxid und Wasserstoff als Syntheseedukte vorgeschlagen, wobei die Mischung dadurch gekennzeichnet ist, dass diese besteht aus
I) einer Komponente A) in Form von wenigstens einem Salz, das gebildet ist aus dem Bis(trifluormethylsulfonyl)imid-Anion 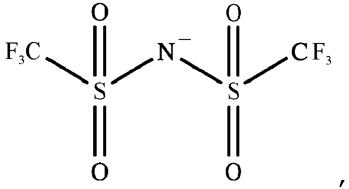 und einem Kation in Form von einem
   - quaternären Ammonium-Kation der allgemeinen Formel [NR¹R²R³R]⁺; oder
   - einem Phosphonium-Kation der allgemeinen Formel [PR¹R²R³R]⁺;
   - wobei die Reste R¹, R², R³ unabhängig voneinander ausgewählt sind aus Wasserstoff; linearen oder verzweigten, gesättigten oder ungesättigten, aliphatischen oder alicyclischen Alkylgruppen mit 1 bis 20 Kohlenstoffatomen, wobei bis zu 6 Wasserstoffreste durch OH-Gruppen substituiert sein können; Heteroaryl-Gruppen und/oder Heteroaryl-C₁-C₆-Alkylgruppen mit 3 bis 8 Kohlenstoffatomen, bevorzugt 4 bis 8 Kohlenstoffatomen im Heteroaryl-Rest und wenigstens einem Heteroatom im Heteroaryl-Rest, das ausgewählt ist aus N, 0 und S, wobei die Alkyl-Gruppen linear oder verzweigt, gesättigt oder ungesättigt, aliphatisch oder alicyclisch sein können und wobei ein oder mehrere Wasserstoffreste am Heteroaryl-Rest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; Aryl- und/oder Aryl-C₁-C₆-Alkylgruppen mit 6 bis 20 Kohlenstoffatomen im Arylrest, wobei die Alkylgruppen linear oder verzweigt, gesättigt oder ungesättigt, aliphatisch oder alicyclisch sein können und wobei am Arylrest ein oder mehrere Wasserstoffreste substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; und
   - [-(CH₂)ₓ-O-]_{y}-CH₃-Gruppen mit x=2-5 und y=1-20; und
   - der Rest R ausgewählt ist aus linearen oder verzweigten, gesättigten oder ungesättigten, aliphatischen oder alicyclischen Alkylgruppen mit 1 bis 20 Kohlenstoffatomen, wobei bis zu 6 Wasserstoffreste durch OH-Gruppen substituiert sein können; Heteroaryl-C₁-C₆-Alkylgruppen mit 3 bis 8 Kohlenstoffatomen, bevorzugt 4 bis 8 Kohlenstoffatomen im Heteroarylrest und wenigstens einem Heteroatom im Heteroaryl-Rest, das ausgewählt ist aus N, 0 und S, wobei die Alkylgruppen linear oder verzweigt, gesättigt oder ungesättigt, aliphatisch oder alicyclisch sein können und wobei ein oder mehrere Wasserstoffreste am Heteroarylrest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; Aryl-C₁-C₆-Alkylgruppen mit 6 bis 20 Kohlenstoffatomen im Arylrest, wobei die Alkylgruppen linear oder verzweigt, gesättigt oder ungesättigt, aliphatisch oder alicyclisch sein können und wobei ein oder mehrere Wasserstoffreste am Arylrest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; und -[-(CH₂)ₓ-O-]_{y}-CH₃-Gruppen mit x=2-5 und y=1-20;
   und
II) einer Komponente B), die aus wenigstens der nachfolgenden Komponente besteht:
   B3) einer zwitterionischen Verbindung, die gebildet ist aus einem der in A) genannten Kationen, bei denen einer der Reste R, R¹, R² oder R³ eine -(CH₂)ₓ-SO₃⁻-Gruppe mit x = 1-10 ist.

Bei der Komponente B3) umfassen die Reste R, R¹, R² oder R³ alle diejenigen, die in Bezug auf die Komponente A) genannt sind, wobei zusätzlich ein jeder der Reste R, R¹, R² oder R³ auch eine -(CH₂)ₓ-SO₃⁻-Gruppe mit x = 1-10 sein kann und einer der Reste R, R¹, R² oder R³ auch tatsächlich eine -(CH₂)ₓ-SO₃⁻-Gruppe mit x = 1-10 ist.

Die Komponente B) kann weiterhin aus wenigstens einer der nachfolgenden Komponenten bestehen:
B1) einem Salz, das gebildet ist aus einem der Anionen [PO₄]³⁻, [HPO₄]²⁻, [H₂PO₄]⁻, [SO₄]²⁻, [HSO₄]⁻, [NO₃]⁻, [NO₂]⁻ oder Cl⁻ und einem, zwei oder drei der in A) genannten Kationen, wobei die Anzahl an Kationen dem absoluten Betrag der Ladungszahl des jeweiligen Anions entspricht;
B2) einem Salz, das gebildet ist aus einem oder mehreren Bis(trifluormethylsulfonyl)imid-Anionen 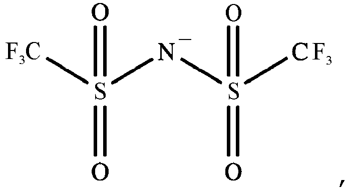 und einem Lithium-, Kalium-, Cäsium-, Magnesium-, Kalzium-, Barium-, Nickel-, Cobalt-, Eisen-, Scandium, Lanthan, Zink, Gallium, Cer oder Aluminium-Kation, wobei die Anzahl der Bis(trifluormethylsulfonyl)imid-Anionen dem absoluten Betrag der Ladungszahl des jeweiligen Metallkations entspricht.

Die Mischung gemäß der vorliegenden Erfindung kann somit eine oder mehrere der Komponenten A) und eine oder mehrere der Komponenten B1, B2 und/oder B3 aufweisen.

Wie von den Erfindern herausgefunden wurde, sind organische Salze mit einem organischen Kation und dem Bis(trifluormethylsulfonyl)imid-Anion jedenfalls bei den bei der Synthese von Methanol aus Kohlenmonoxid und Wasserstoff, Kohlendioxid und Wasserstoff oder einer Mischung aus Kohlenmonoxid, Kohlendioxid und Wasserstoff gegebenen Reaktionsbedingungen (Temperaturen im Bereich von 200°C bis 300°C und Drücken im Bereich von 50 bar bis 300 bar) flüssig und ausreichend stabil.

Diese organischen Salze weisen jedoch - bedingt durch die Natur des Anions - stets sehr hydrophobe, also sehr geringe Wasser- und/oder Methanol-lösende Eigenschaften auf. Durch die geringe Löslichkeit dieser Reaktionsprodukte wird der gewünschte Effekt einer Gleichgewichtsverschiebung durch Einlösen von Wasser und/oder Methanol in die ionische Flüssigkeit bei der Methanol-Synthese nicht in ausreichendem Maße erreicht.

Die (zusätzliche) Verwendung von einer oder mehreren bekannten Wasser- und/oder Methanol-lösenden, organischen ionischen Flüssigkeit(en) scheidet aus, da dies aufgrund von deren thermischer Instabilität zu gasförmigen Zersetzungsprodukten führen würde, die den eingesetzten Katalysator deaktivieren.

Überraschend wurde nun gefunden, dass durch eine Mischung aus wenigstens einer der oben angeführten Komponenten A (d.h. einem thermisch stabilen, organischen Bis(trifluormethylsulfonyl)imid-Salz) und wenigstens der oben angeführten Komponente B3 (zwitterionische Verbindung) stärker Wasser- und Methanol-lösende, thermisch stabile, niedrigschmelzende Sorptionsphasen erhalten werden, die erfolgreich in der Methanol-Synthese aus Kohlendioxid (CO₂) und/oder Kohlenmonoxid (CO) mit Wasserstoff (H₂) eingesetzt werden können.

Wie Experimente ergeben haben, kann bspw. bei der Methanol-Synthese unter Verwendung von Kohlendioxid und Wasserstoff durch Verwendung einer erfindungsgemäßen Mischung als flüssiges Sorptionsmittel durch Sorption der Reaktionsprodukte Wasser und Methanol aus der gasförmigen Reaktionsphase eine Gleichgewichtsverschiebung um einen Faktor 4 und mehr erreicht werden, d.h. es wird im geschlossenen Reaktor mehr als viermal mehr Methanol gebildet, als dies unter Gleichgewichtsbedingungen ohne die erfindungsgemäße Mischung möglich wäre.

Gemäß einer ersten vorteilhaften Weiterbildung der Mischung beträgt der Masseanteil der Komponente B), d.h. der Summe der Masseanteile in der ionischen Flüssigkeit im Bereich von 1% bis 99%, bevorzugt im Bereich von 1% und 80%.

Wie von den Erfindern ebenfalls überraschend herausgefunden wurde, ist oftmals bereits ein vergleichsweise kleiner Masseanteil der Komponente B) ausreichend, um eine deutlich verbesserte Wasser- und/oder Methanol-lösende Eigenschaft der Mischung im Vergleich zur Komponente A) allein zu erreichen. Welcher Masseanteil an der jeweiligen Komponente B) in der Mischung mit der jeweiligen Komponente A) die erforderlichen oder gewünschten Lösungseigenschaften gegenüber Wasser und/oder Methanol bewirkt, kann von einem Fachmann durch einige wenige Versuche ermittelt werden.

Gemäß einer weiteren vorteilhaften Weiterbildung ist die erfindungsgemäße Mischung eine, die bei einer Temperatur ab 79°C, bevorzugt ab 49°C, besonders bevorzugt ab 19°C flüssig ist. Da die Mischungen gemäß der vorliegenden Erfindung als flüssige Sorptionsphase vorgesehen sind, ist es jedenfalls erforderlich, dass diese bei den Temperaturbedingungen, die während des Sorptionsvorgangs gegeben sind, flüssig sind.

Findet der Sorptionsvorgang bspw. während der Gasphasen-Methanol-Synthese statt ("in-situ"), bei der Reaktionstemperaturen im Bereich von etwa 100°C bis etwa 300°C gegeben sind, muss eine Mischung gewählt werden, die in diesem Temperaturbereich flüssig ist. Findet der Sorptionsvorgang bspw. außerhalb des Reaktors statt ("ex-situ"), wobei hier niedrigere Temperaturen als im Reaktor gegeben sein können, oder findet der Sorptionsvorgang in einem Bereich des Reaktors statt, in dem niedrigere Temperaturen herrschen als in der eigentlichen "Reaktionszone", muss eine Mischung gewählt werden, die bei den gegebenen niedrigeren Temperaturen flüssig ist. Eine oder mehrere jeweils geeignete Mischung kann ein Fachmann durch einige wenige Versuche ermitteln.

Wie von den Erfindern herausgefunden wurde, weisen in vielen Fällen die beanspruchten Mischungen einen Schmelzpunkt jedenfalls von unter 80°C auf, wenn die Komponente A) in einem Masseverhältnis von 20% oder mehr in der Mischung vorliegt. Der jeweilige Schmelzpunkt einer Mischung kann durch einen einfachen Versuch ermittelt werden und es können die Komponenten der Mischung und deren Mischungsverhältnisse (Massenverhältnisse) so gewählt werden, dass sowohl ein gewünschter oder erforderliche Schmelzpunkt als auch eine gewünschter Grad an Methanol- und/oder Wasser-lösender Eigenschaft erreicht wird.

Die Erfinder konnten zeigen, dass in vielen Fällen über einen gewissen Masseanteil-Bereich der Komponente B) eine lineare Beziehung zwischen der Zunahme an Methanol- und/oder Wasser-lösender Eigenschaft und der Zunahme des Masseanteils der Komponente B) existiert.

Die erfindungsgemäßen Mischungen werden durch intensives Mischen der sie enthalten Komponenten erhalten. Die einzelnen Komponenten sind allesamt durch Fachleuten bekannte Verfahren herstellbar und sind auch kommerziell (gegebenenfalls auf gesonderte Bestellung hin) erhältlich.

Sofern in der vorliegenden Anmeldung davon die Rede ist, dass eine Alkyl-, Alkoxy- und Aminoalkyl-Gruppe verzweigt sein kann, so setzt dies selbstverständlich voraus, dass die Gruppe die erforderliche Mindestanzahl von 3 Kohlenstoffatomen aufweist; sofern in der vorliegenden Anmeldung davon die Rede ist, dass eine Gruppe ungesättigt ist, so setzt dies selbstverständlich voraus, dass diese wenigstens 2 Atome aufweist, die jedenfalls eine Doppelbindung miteinander ausbilden können (etwa mindestens 2 Kohlenstoffatome); sofern in der vorliegenden Anmeldung davon die Rede ist, dass eine Gruppe eine alicyclische ist, so setzt dies selbstverständlich voraus, dass die Gruppe wenigstens 3, bevorzugt 4, 5, 6 oder mehr Kohlenstoffe aufweist; sofern in der vorliegenden Anmeldung die Rede ist, dass bei einer Gruppe bis zu 6 Wasserstoffreste durch OH-Gruppen substituiert sein können, so setzt dies selbstverständlich voraus, dass die Gruppe über die erforderliche Anzahl an substituierbaren Wasserstoffresten verfügt (bei einer C₁-Gruppe können maximal 3 Wasserstoffreste substituiert sein; bei einer gesättigten C₂-Gruppe maximal 5 Wasserstoffreste; etc.). Sofern in der vorliegenden Anmeldung die Rede ist von einem Aryl-Rest mit 6 bis 20 Kohlenstoffen, so kann es sich dabei in Abhängigkeit von der Anzahl an Kohlenstoffen um einen monocyclischen (bspw. mit bis zu 10 Kohlenstoffatomen) oder polycyclischen (bspw. ab 10 Kohlenstoffatomen) Aryl-Rest handeln, wobei die Ringe eines polycyclischen Aryl-Rests kondensiert sein können oder mittels einer C-C-Bindung (wie bspw. beim Biphenyl) miteinander verbunden sein können. Und sofern in der vorliegenden Anmeldung die Rede ist von "Halogenatomen", so sind darunter Fluor-, Chlor-, Brom- und/oder Iod-Atome bzw. -Reste zu verstehen.

Im nachfolgenden ist ein bevorzugtes Beispiel der Mischung angegeben:
Tributylmethylphosphonium-bis(trifluormethylsulfonyl)imid, Lithium-bis(trifluormethylsulfonyl)imid und Tributyl-4-sulfonyl-1-butanphosphonium.

Bei der angegebenen Mischung beträgt das Massenverhältnis der ersten angegebenen Komponente zu der zweiten angegebenen Komponente zu der dritten angegebenen Komponente bevorzugt im Bereich von vier zu eins zu eins über vier zu drei zu eins bis vier zu drei zu drei. Derartige Massenverhältnisse stellen regelmäßig auch bei anderen binären und ternären Mischungen gemäß der vorliegenden Erfindung geeignete Massenverhältnisse dar.

Selbstverständlich ist die vorliegende Erfindung nicht auf die oben angeführten Beispiele und die angegebenen bevorzugten Massenverhältnisse beschränkt; vielmehr ergibt sich der Schutzumfang der vorliegenden Erfindung aus dem Inhalt der Patentansprüche.

Von der vorliegenden Erfindung nicht umfasst ist die Verwendung der Mischungen gemäß der vorliegenden Erfindung bei einer Methanol-Synthese, insbesondere einer Gasphasen-Methanol-Synthese, als flüssiges Sorptionsmittel für Methanol bzw. Methanol und Wasser.

Von der vorliegenden Erfindung umfasst ist auch ein Verfahren zur Durchführung einer Methanol-Synthese, insbesondere einer Gasphasen-Methanol-Synthese, unter Verwendung von Kohlenmonoxid und Wasserstoff, Kohlendioxid und Wasserstoff oder einer Mischung aus Kohlenmonoxid, Kohlendioxid und Wasserstoff als Syntheseedukte in einem Reaktor bei einer Temperatur im Bereich von 100°C und 300°C und einem Synthesegasdruck im Bereich von 50 bar bis 300 bar in Gegenwart eines Katalysators, umfassend folgende Schritte:
- Bereitstellen einer erfindungsgemäßen flüssigen Mischung oder einer ihrer vorteilhaften Weiterbildungen in dem Reaktor;
- Umsetzen von Kohlenmonoxid und Wasserstoff, Kohlendioxid und Wasserstoff oder einer Mischung von Kohlenmonoxid, Kohlendioxid und Wasserstoff zu dem Syntheseprodukt Methanol oder den Syntheseprodukten Methanol und Wasser in dem Reaktor bei einer Temperatur im Bereich von 100°C und 300°C und einem Synthesegasdruck im Bereich von 50 bar bis 300 bar;
- Sorption von wenigstens einer Teilmenge von wenigstens einem Syntheseprodukt aus der wenigstens ein Syntheseprodukt enthaltenden Gasphase in die flüssige Mischung; und
- kontinuierliches oder diskontinuierliches Ausleiten der flüssigen Mischung aus dem Reaktor.

Bezüglich des Reaktors bestehen keine besonderen Einschränkungen und es kann jeder Reaktor verwendet werden, der für den angegebenen Zweck aus dem Stand der Technik bekannt ist, bspw. auch einer, wie er in der DE 10 2015 202 681 A1 beschrieben ist. Diesbezüglich und auch bezüglich der möglichen Reaktionsführung wird ausdrücklich auch auf diese Druckschrift Bezug genommen.

In den Reaktor kann eine geeignete Menge der erfindungsgemäßen Mischung oder einer ihrer vorteilhaften Weiterbildungen eingebracht werden, wobei der Reaktor anschließend auf eine für die Methanol-Synthese geeignete Temperatur im Bereich von 100°C bis 300°C (bspw. im Bereich von 200°C) aufgeheizt werden kann oder bereits bei Einbringung der Mischung eine hierfür geeignete Temperatur aufweisen kann. Hierdurch liegt die erfindungsgemäße Mischung in dem Reaktor in jedem Fall in flüssiger Form vor. Die Mischung kann selbstverständlich auch bereits außerhalb des Reaktors eine Temperatur aufweisen, bei der die Mischung flüssig ist, und die Mischung kann in flüssiger Form in den Reaktor eingebracht werden.

Oberhalb der Mischung kann in dem Reaktor der für die Methanol-Synthese erforderliche, pelletierte oder partikelförmige Katalysator angeordnet sein, etwa in einem korbähnlichen Behälter. Durch Einleiten der Synthesegase Kohlenmonoxid und Wasserstoff, Kohlendioxid und Wasserstoff oder einer Mischung aus Kohlenmonoxid, Kohlendioxid und Wasserstoff in den Reaktor und Einstellung des Synthesegasdrucks auf einen Bereich von 50 bar bis 300 bar (bspw. im Bereich von 80 bar) und einer Reaktortemperatur im Bereich von 100°C bis 300°C (bspw. 200°C) findet die Umsetzung der Synthesegas-Edukte zu dem/den Synthesegas-Produkt(en) Methanol bzw. Methanol und Wasser statt.

Durch die Methanol- bzw. Methanol- und Wasser-lösende Eigenschaft der flüssigen Mischung gemäß der vorliegenden Erfindung findet ein Übertritt von wenigstens einer Teilmenge des Syntheseprodukts Methanol oder der Syntheseprodukte Methanol und Wasser in die Mischung statt, wodurch das Gleichgewicht in den obigen Reaktionsgleichungen 1) und 2) in Richtung der Edukte verschoben wird. Somit kann in dem Reaktor ein viel größerer Anteil an Edukten in das/die Produkt(e) umgesetzt werden, als dies ohne die Anwesenheit der Mischung gemäß der vorliegenden Erfindung möglich wäre.

Ist eine ausreichende, gewünschte oder die maximal mögliche Menge des Reaktionsprodukts/der Reaktionsprodukte in die flüssige Mischung übergetreten, wird diese aus dem Reaktor ausgeleitet.

Bei dem Verfahren gemäß der vorliegenden Erfindung kann in vorteilhafter Weise während des Vorgangs der Methanol-Synthese oder nach dem Vorgang der Methanol-Synthese die wenigstens ein Syntheseprodukt enthaltende Gasphase mittels eines Gaseintragrührers in die flüssige Mischung eingetragen werden. Hierdurch kann die Sorption des Reaktionsprodukts Methanol bzw. der Reaktionsprodukte Methanol und Wasser im Vergleich ohne eine solche Maßnahme beschleunigt erfolgen. Selbstverständlich kann eine Sorption aus der Gasphase des Syntheseprodukts/der Syntheseprodukte auch durch andere geeignete Maßnahmen beschleunigt werden, etwa indem die Gasphase in die flüssige Mischung eingeblasen wird.

Das Verfahren gemäß der vorliegenden Erfindung kann in vorteilhafter Weise auch dahin weitergebildet sein, dass die aus dem Reaktor ausgeleitete flüssige Mischung entspannt wird und dass wenigstens das eine dadurch zumindest teilweise aus der Mischung austretende Syntheseprodukt Methanol gewonnen wird. Aus einer ausreichend heißen bzw. warmen, flüssigen Mischung tritt das Syntheseprodukt Methanol bzw. treten die Syntheseprodukte Methanol und Wasser gasförmig aus und kann/können dann bspw. durch Abkühlung kondensiert werden.

Ebenfalls von Vorteil ist es, wenn bei dem Verfahren gemäß der vorliegenden Erfindung nach der Entspannung der flüssigen Mischung und dem zumindest teilweisen Austreten des Syntheseprodukts Methanol oder der Syntheseprodukte Methanol und Wasser aus der Mischung die Mischung wieder in den Reaktor zurückgeführt wird. Die Rückführung der Mischung geschieht bevorzugt in einem flüssigen Zustand der Mischung. Die Rückführung der Mischung in den Reaktor kann - wie auch deren Ausleitung aus dem Reaktor - kontinuierlich oder diskontinuierlich erfolgen. Gleiches gilt für die Zuführung der SyntheseEdukte.

Somit kann durch die vorliegende Erfindung eine kontinuierliche Methanol-Synthese durchgeführt werden, wobei wenigstens Teilmengen des Syntheseprodukts Methanol bzw. der Syntheseprodukte Methanol und Wasser in die flüssige Mischung überführt und kontinuierlich oder diskontinuierlich aus dem Reaktor ausgeführt werden können. Außerhalb des Reaktors kann die Mischung entspannt werden, das/die austretenden Syntheseprodukt(e) gewonnen und die von dem/den Syntheseprodukt(en) zumindest teilweise befreite Mischung zur erneuten Sorption von Syntheseprodukt(en) in den Reaktor zurückgeführt werden.

Bei dem Verfahren gemäß der vorliegenden Erfindung kann jeder geeignete Katalysator verwendet werden, bevorzugt wird jedoch Kupfer-Zink-Oxid auf Aluminium-Oxid als Katalysator verwendet, der in der Gasphase des Reaktors angeordnet ist.

Ein bevorzugtes Beispiel des Verfahrens gemäß der vorliegenden Erfindung ist eines, bei dem als Synthesegas Kohlendioxid und Wasserstoff verwendet wird, im Reaktor wenigstens Teilmengen der Syntheseprodukte Methanol und Wasser in die flüssige Mischung sorbiert werden, nach dem Ausleiten der Mischung aus dem Reaktor und dem Entspannen der Mischung (d.h. Verminderung des Drucks über der Mischung) die Syntheseprodukte Methanol und Wasser zumindest teilweise aus der Mischung austreten, wenigstens das ausgetretene Syntheseprodukt Methanol (etwa durch Kondensation) gewonnen wird, und nach dem Entspannen der flüssigen Mischung und dem zumindest teilweisen Austreten der Syntheseprodukte aus der Mischung diese (bevorzugt in flüssiger Form) wieder in den Reaktor zurückgeführt wird.

Das Verfahren gemäß der vorliegenden Erfindung kann in vorteilhafter Weise auch dahin weitergebildet sein, dass der Mischung vor dem Rückführen in den Reaktor Wärme entzogen wird, d.h. dass die Temperatur der Mischung abgesenkt wird.

Die Mischungen gemäß der vorliegenden Erfindung können im flüssigen Zustand als Sorptionsmittel für Methanol und/oder Wasser verwendet werden und zwar für alle Verfahren, bei denen eine solche Sorption gewünscht ist, also nicht nur im Rahmen einer Methanol-Gasphasen-Synthese. Eine derzeit bevorzugte Anwendung ist deren Verwendung "in-situ", d.h. bei der Methanol-Gasphasen-Synthese, jedoch ist die Verwendung nicht auf eine solche beschränkt. Eine Sorption von Methanol und/ oder Wasser kann unter allen geeigneten Druck- (bspw. auch bei Atmosphärendruck) und Temperaturbedingungen (d.h. ab einer Temperatur, bei der die jeweilige Mischung flüssig ist) stattfinden bzw. durchgeführt werden.

Soweit in der vorliegenden Anmeldung der Begriff "Sorptionsmittel" verwendet wird, so ist darunter gemäß der allgemein anerkannten Definition ein Mittel zu verstehen, das geeignet ist, eine Anreicherung von wenigstens einem Stoff (hier: Methanol und/oder Wasser) innerhalb einer Phase (hier: der erfindungsgemäßen, flüssigen Mischung oder einer ihrer vorteilhaften Weiterbildungen) oder auf einer Grenzfläche zwischen zwei Phasen (hier: auf der Grenzfläche, die gebildet ist zwischen einer Methanol und/oder Wasser enthaltenden Gasphase und der flüssigen Phase der erfindungsgemäßen, flüssigen Mischung oder einer ihrer vorteilhaften Weiterbildungen) zu bewirken. Ein Sorptionsmittel kann somit eine Anreicherung von wenigstens einem Stoff innerhalb einer Phase (d.h. eine Absorption) und/oder eine Anreicherung an einer Grenzfläche (d.h. eine Adsorption) bewirken, wobei bei den Mischungen gemäß der vorliegenden Erfindung bei deren Verwendung als flüssige Sorptionsmittel davon auszugehen ist, dass ausschließlich oder doch ganz überwiegend eine Absorption von Methanol und/oder Wasser erfolgt.

## Patentansprüche

1. Mischung zur Verwendung als flüssiges Sorptionsmittel für Methanol oder Methanol und Wasser bei der Methanol-Synthese unter Verwendung von Kohlenmonoxid und Wasserstoff, Kohlendioxid und Wasserstoff oder einer Mischung aus Wasserstoff, Kohlenmonoxid und Kohlendioxid als Syntheseedukte, **dadurch gekennzeichnet, dass** die Mischung besteht aus
I) einer Komponente A) in Form von wenigstens einem Salz, das gebildet ist aus dem Bis(trifluormethylsulfonyl)imid-Anion 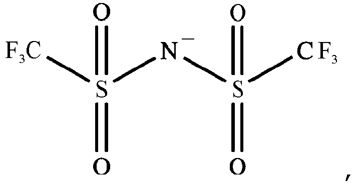 und einem Kation in Form von einem
- quaternären Ammonium-Kation der allgemeinen Formel [NR¹R²R³R]⁺; oder
- einem Phosphonium-Kation der allgemeinen Formel [PR¹R²R³R]⁺; wobei die Reste R¹, R², R³ unabhängig voneinander ausgewählt sind aus Wasserstoff; linearen oder verzweigten, gesättigten oder ungesättigten, aliphatischen oder alicyclischen Alkylgruppen mit 1 bis 20 Kohlenstoffatomen, wobei bis zu 6 Wasserstoffreste durch OH-Gruppen substituiert sein können; Heteroaryl-Gruppen und/oder Heteroaryl-C₁-C₆-Alkylgruppen mit 3 bis 8 Kohlenstoffatomen im Heteroaryl-Rest und wenigstens einem Heteroatom im Heteroaryl-Rest, das ausgewählt ist aus N, 0 und S, wobei die Alkyl-Gruppen linear oder verzweigt, gesättigt oder ungesättigt, aliphatisch oder alicyclisch sein können und wobei ein oder mehrere Wasserstoffreste am Heteroaryl-Rest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; Aryl- und/oder Aryl-C₁-C₆-Alkylgruppen mit 6 bis 20 Kohlenstoffatomen im Arylrest, wobei die Alkylgruppen linear oder verzweigt, gesättigt oder ungesättigt, aliphatisch oder alicyclisch sein können und wobei am Arylrest ein oder mehrere Wasserstoffreste substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; und -[-(CH₂)ₓ-O-]_{y}-CH₃-Gruppen mit x=2-5 und y=1-20; und
- der Rest R ausgewählt ist aus linearen oder verzweigten, gesättigten oder ungesättigten, aliphatischen oder alicyclischen Alkylgruppen mit 1 bis 20 Kohlenstoffatomen, wobei bis zu 6 Wasserstoffreste durch OH-Gruppen substituiert sein können; Heteroaryl-C₁-C₆-Alkylgruppen mit 3 bis 8 Kohlenstoffatomen im Heteroarylrest und wenigstens einem Heteroatom im Heteroaryl-Rest, das ausgewählt ist aus N, 0 und S, wobei die Alkylgruppen linear oder verzweigt, gesättigt oder ungesättigt, aliphatisch oder alicyclisch sein können und wobei ein oder mehrere Wasserstoffreste am Heteroarylrest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; Aryl-C₁-C₆-Alkylgruppen mit 6 bis 20 Kohlenstoffatomen im Arylrest, wobei die Alkylgruppen linear oder verzweigt, gesättigt oder ungesättigt, aliphatisch oder alicyclisch sein können und wobei ein oder mehrere Wasserstoffreste am Arylrest substituiert sein können mit wenigstens einer Gruppe, die unabhängig voneinander auswählbar ist aus linearen oder verzweigten, gesättigten oder ungesättigten, aliphatischen oder alicyclischen C₁-C₆-Alkylgruppen und/oder Halogenatomen; und -[-(CH₂)ₓ-O-]_{y}-CH₃-Gruppen mit x=2-5 und y=1-20;
und
II) einer Komponente B), die aus wenigstens der nachfolgenden Komponenten besteht:
B3) einer zwitterionischen Verbindung, die gebildet ist aus einem der in A) genannten Kationen, bei denen einer der Reste R, R¹, R² oder R³ eine -(CH₂)ₓ-SO₃⁻-Gruppe mit x = 1-10 ist.

2. Komponente B) gemäß Anspruch 1, weiterhin bestehend aus wenigstens einer der nachfolgenden Komponenten:
B1) einem Salz, das gebildet ist aus einem der Anionen [PO₄]³⁻, [HPO₄]²⁻, [H₂PO₄]⁻, [SO₄]²⁻, [HSO₄]⁻, [NO₃]⁻, [NO₂]⁻ oder Cl⁻ und einem, zwei oder drei der in A) genannten Kationen, wobei die Anzahl an Kationen dem absoluten Betrag der Ladungszahl des jeweiligen Anions entspricht;
B2) einem Salz, das gebildet ist aus einem oder mehreren Bis(trifluormethylsulfonyl)imid-Anionen 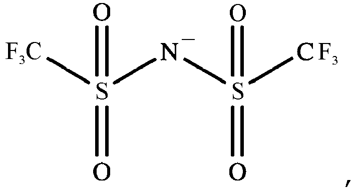 und einem Lithium-, Kalium-, Cäsium-, Magnesium-, Kalzium-, Barium-, Nickel-, Cobalt-, Eisen-, Scandium, Lanthan, Zink, Gallium, Cer oder Aluminium-Kation, wobei die Anzahl der Bis(trifluormethylsulfonyl)imid-Anionen dem absoluten Betrag der Ladungszahl des jeweiligen Metallkations entspricht.

3. Mischung gemäß Anspruch 1 oder Anspruch 2, **dadurch gekennzeichnet, dass** der Masseanteil der Komponente B) in der Mischung im Bereich von 1% bis 99% beträgt, bevorzugt im Bereich von 1% und 80%.

4. Mischung gemäß einem der vorhergehenden Ansprüche, **dadurch gekennzeichnet, dass** diese bei einer Temperatur ab 79°C, bevorzugt ab 49°C, besonders bevorzugt ab 19°C flüssig ist.

5. Mischung gemäß einem der vorhergehenden Ansprüche, **dadurch gekennzeichnet, dass** sie besteht aus Tributylmethylphosphonium-bis(trifluormethylsulfonyl)imid, Lithium-bis(trifluormethylsulfonyl)imid und Tributyl-4-sulfonyl-1-butanphosphonium

6. Mischung gemäß Anspruch 5, **dadurch gekennzeichnet, dass** das Massenverhältnis der ersten angegebenen Komponente zu der zweiten angegebenen Komponente zu der dritten angegebenen Komponente im Bereich von vier zu eins zu eins über vier zu drei zu eins bis vier zu drei zu drei beträgt.

7. Verfahren zur Durchführung einer Methanol-Synthese unter Verwendung von Kohlenmonoxid und Wasserstoff, Kohlendioxid und Wasserstoff oder einer Mischung aus Kohlenmonoxid, Kohlendioxid und Wasserstoff als Synthesedukte in einem Reaktor bei einer Temperatur im Bereich von 100°C und 300°C und einem Synthesegasdruck im Bereich von 50 bar bis 300 bar in Gegenwart eines Katalysators, umfassend folgende Schritte:
- Bereitstellen einer flüssigen Mischung gemäß einem der Ansprüche 1 bis 6 in dem Reaktor;
- Umsetzen von Kohlenmonoxid und Wasserstoff, Kohlendioxid und Wasserstoff oder eine Mischung aus Kohlenmonoxid, Kohlendioxid und Wasserstoff zu dem Syntheseprodukt Methanol oder den Syntheseprodukten Methanol und Wasser in dem Reaktor bei einer Temperatur im Bereich von 100°C und 300°C und einem Synthesegasdruck im Bereich von 50 bar bis 300 bar;
- Sorption von wenigstens einer Teilmenge von wenigstens einem Syntheseprodukt aus der wenigstens ein Syntheseprodukt enthaltenden Gasphase in die flüssige Mischung; und
- kontinuierliches oder diskontinuierliches Ausleiten der flüssigen Mischung aus dem Reaktor.

8. Verfahren gemäß Anspruch 7, **dadurch gekennzeichnet, dass** die wenigstens ein Syntheseprodukt enthaltende Gasphase mittels eines Gaseintragrührers in die Mischung eingetragen wird.

9. Verfahren gemäß Anspruch 7 oder 8, **dadurch gekennzeichnet, dass** die aus dem Reaktor ausgeleitete flüssige Mischung entspannt wird und dass wenigstens das dadurch zumindest teilweise aus der Mischung austretende Methanol gewonnen wird.

10. Verfahren nach Anspruch 9, **dadurch gekennzeichnet, dass** nach der Entspannung der flüssigen Mischung und dem zumindest teilweisen Austreten von dem Syntheseprodukt Methanol oder den Syntheseprodukten Methanol und Wasser aus der Mischung die Mischung wieder in den Reaktor zurückgeführt wird.

11. Verfahren nach einem der Ansprüche 7 bis 10, **dadurch gekennzeichnet, dass** als Katalysator Kupfer-Zink-Oxid auf Aluminium-Oxid verwendet wird, der in der Synthesegas-Gasphase des Reaktors angeordnet ist.

12. Verfahren nach einem der Ansprüche 7 bis 11, **dadurch gekennzeichnet, dass** als Synthesegas Kohlendioxid und Wasserstoff verwendet wird, im Reaktor wenigstens Teilmengen der Syntheseprodukte Methanol und Wasser in die flüssige Mischung absorbiert werden, nach dem Ausleiten der Mischung aus dem Reaktor und dem Entspannen der Mischung die Syntheseprodukte Methanol und Wasser zumindest teilweise aus der Mischung austreten, wenigstens das zumindest teilweise ausgetretene Syntheseprodukt Methanol gewonnen wird, und nach dem Entspannen der flüssigen Mischung und dem zumindest teilweise Austreten der Syntheseprodukte aus der Mischung diese wieder in den Reaktor zurückgeführt wird.

13. Verfahren nach einem der Ansprüche 10 bis 12, **dadurch gekennzeichnet, dass** der Mischung vor dem Rückführen in den Reaktor Wärme entzogen wird.

## Claims

1. Mixture for use as liquid sorbent for methanol or methanol and water in methanol synthesis using carbon monoxide and hydrogen, carbon dioxide and hydrogen or a mixture of hydrogen, carbon monoxide and carbon dioxide as synthesis reactants, **characterized in that** the mixture consists of
I) a component A) in the form of at least one salt, which is formed from the bis(trifluoromethylsulfonyl)imide anion 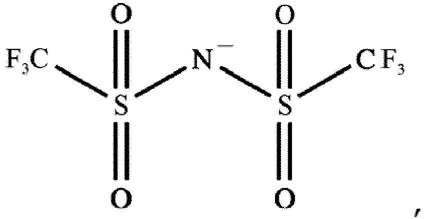 and a cation in the form of a
- quaternary ammonium cation of the general formula [NR¹R²R³R]⁺; or
- a phosphonium cation of the general formula [PR¹R²R³R]⁺;
wherein the radicals R¹, R², R³ are independently selected from hydrogen; linear or branched, saturated or unsaturated, aliphatic or alicyclic alkyl groups having 1 to 20 carbon atoms, where up to 6 hydrogen radicals may be substituted by OH groups; heteroaryl groups and/or heteroaryl-C₁-C₆-alkyl groups having 3 to 8 carbon atoms in the heteroaryl radical and at least one heteroatom in the heteroaryl radical that is selected from N, O and S, wherein the alkyl groups may be linear or branched, saturated or unsaturated, aliphatic or alicyclic and wherein one or more hydrogen radicals on the heteroaryl radical may be substituted by at least one group which is independently selectable from linear or branched, saturated or unsaturated, aliphatic or alicyclic C₁-C₆-alkyl groups and/or halogen atoms; aryl and/or aryl-C₁-C₆-alkyl groups having 6 to 20 carbon atoms in the aryl radical, wherein the alkyl groups may be linear or branched, saturated or unsaturated, aliphatic or alicyclic and wherein on the aryl radical one or more hydrogen radicals may be substituted by at least one group which is independently selectable from linear or branched, saturated or unsaturated, aliphatic or alicyclic C₁-C₆-alkyl groups and/or halogen atoms; and - [-(CH₂)ₓ-O-]_{y}-CH₃ groups where x = 2-5 and y = 1-20; and
- the radical R is selected from linear or branched, saturated or unsaturated, aliphatic or alicyclic alkyl groups having 1 to 20 carbon atoms, where up to 6 hydrogen radicals may be substituted by OH groups; heteroaryl-C₁-C₆-alkyl groups having 3 to 8 carbon atoms in the heteroaryl radical and at least one heteroatom in the heteroaryl radical that is selected from N, O and S, wherein the alkyl groups may be linear or branched, saturated or unsaturated, aliphatic or alicyclic and wherein one or more hydrogen radicals on the heteroaryl radical may be substituted by at least one group which is independently selectable from linear or branched, saturated or unsaturated, aliphatic or alicyclic C₁-C₆-alkyl groups and/or halogen atoms; aryl-C₁-C₆-alkyl groups having 6 to 20 carbon atoms in the aryl radical, wherein the alkyl groups may be linear or branched, saturated or unsaturated, aliphatic or alicyclic and wherein one or more hydrogen radicals on the aryl radical may be substituted by at least one group which is independently selectable from linear or branched, saturated or unsaturated, aliphatic or alicyclic C₁-C₆-alkyl groups and/or halogen atoms; and -[-(CH₂)ₓ-O-]_{y}-CH₃ groups where x = 2-5 and y = 1-20;
and
II) a component B) which consists of at least the following components:
B3) a zwitterionic compound that is formed from one of the cations stated in A), in which one of the radicals R, R¹, R² or R³ is a -(CH₂)ₓ-SO₃⁻ group where x = 1-10.

2. Component B) according to Claim 1, additionally composed of at least one of the following components:
B1) a salt that is formed from one of the anions [PO₄]³⁻, [HPO₄]²⁻, [H₂PO₄]⁻, [SO₄]²⁻, [HSO₄]⁻, [NO₃]⁻, [NO₂]⁻ or Cl⁻ and one, two or three of the cations stated in A), wherein the number of cations corresponds to the absolute value of the charge number of the respective anion;
B2) a salt that is formed from one or more bis(trifluoromethylsulfonyl)imide anions 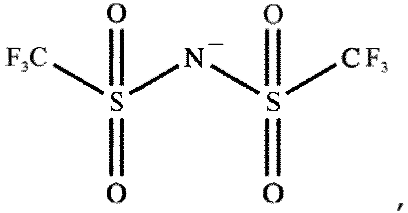 and a lithium, potassium, cesium, magnesium, calcium, barium, nickel, cobalt, iron, scandium, lanthanum, zinc, gallium, cerium or aluminum cation, wherein the number of bis(trifluoromethylsulfonyl)imide anions corresponds to the absolute value of the charge number of the respective metal cation.

3. Mixture according to Claim 1 or Claim 2, **characterized in that** the proportion by mass of component B) in the mixture is in the range from 1% to 99%, preferably in the range from 1% to 80%.

4. Mixture according to any of the preceding claims, **characterized in that** it is liquid at a temperature from 79°C, preferably from 49°C, particularly preferably from 19°C.

5. Mixture according to any of the preceding claims, **characterized in that** it consists of tributylmethylphosphonium bis(trifluoromethylsulfonyl)imide, lithium bis(trifluoromethylsulfonyl)imide and tributyl-4-sulfonyl-1-butanephosphonium.

6. Mixture according to Claim 5, **characterized in that** the mass ratio of the first component specified to the second component specified to the third component specified is in the range from 4:1:1 through 4:3:1 to 4:3:3.

7. Method for conducting a methanol synthesis using carbon monoxide and hydrogen, carbon dioxide and hydrogen or a mixture of carbon monoxide, carbon dioxide and hydrogen as synthesis reactants in a reactor at a temperature in the range from 100°C to 300°C and a synthesis gas pressure in the range from 50 bar to 300 bar in the presence of a catalyst, comprising the following steps:
- providing a liquid mixture according to any of Claims 1 to 6 in the reactor;
- converting carbon monoxide and hydrogen, carbon dioxide and hydrogen or a mixture of carbon monoxide, carbon dioxide and hydrogen to the synthesis product methanol or the synthesis products methanol and water in the reactor at a temperature in the range from 100°C to 300°C and a synthesis gas pressure in the range from 50 bar to 300 bar;
- sorbing at least one subamount of at least one synthesis product, from the gas phase comprising at least one synthesis product, into the liquid mixture; and
- continuously or discontinuously guiding the liquid mixture out of the reactor.

8. Method according to Claim 7, **characterized in that** the gas phase comprising at least one synthesis product is introduced into the mixture by means of a sparging stirrer.

9. Method according to Claim 7 or 8, **characterized in that** the liquid mixture guided out of the reactor is depressurized and **in that** at least the methanol - at least partially emerging from the mixture as a result - is recovered.

10. Method according to Claim 9, **characterized in that**, after the depressurization of the liquid mixture and the at least partial emergence from the mixture of the synthesis product methanol or the synthesis products methanol and water, the mixture is recycled back into the reactor.

11. Method according to any of Claims 7 to 10, **characterized in that** the catalyst used is copper/zinc oxide on aluminum oxide, said catalyst being arranged in the synthesis-gas gas phase of the reactor.

12. Method according to any of Claims 7 to 11, **characterized in that** the synthesis gas used is carbon dioxide and hydrogen; at least subamounts of the synthesis products methanol and water are absorbed into the liquid mixture in the reactor; the guidance of the mixture out of the reactor and the depressurization of the mixture is followed by the synthesis products methanol and water at least partially emerging from the mixture; at least the synthesis product methanol that has at least partially emerged is recovered; and the depressurization of the liquid mixture and the at least partial emergence of the synthesis products from the mixture is followed by the mixture being recycled back into the reactor.

13. Method according to any of Claims 10 to 12, **characterized in that** heat is extracted from the mixture prior to recycling into the reactor.

## Revendications

1. Mélange à utiliser comme agent de sorption liquide du méthanol ou du méthanol et de l'eau dans la synthèse du méthanol en utilisant du monoxyde de carbone et de l'hydrogène, du dioxyde de carbone et de l'hydrogène ou un mélange d'hydrogène, de monoxyde de carbone et de dioxyde de carbone comme éduits de synthèse, **caractérisé en ce que** le mélange est constitué
I) d'un constituant A) sous la forme d'au moins un sel qui est formé de l'anion bis (trifluorométhylsulfonyl) imide 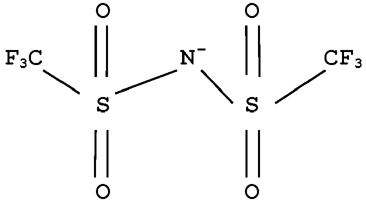 et d'un cation sous la forme d'un
- cation d'ammonium quaternaire de formule brute [NR₁R₂R₃R]+ ou
- cation phosphonium de formule brute [PR₁R₂R₃R+];
les radicaux R₁, R₂, R₃ étant choisis indépendamment les uns des autres parmi l'hydrogène ; des groupes alcoyle linéaires ou ramifiés, saturés ou insaturés, aliphatiques ou alicycliques ayant de 1 à 20 atomes de carbone, jusqu'à 6 radicaux hydrogène pouvant être remplacés par des groupes OH ; des groupes hétéroaryle et/ou des groupes hétéroarylalcoyle ayant de 1 à 6 atomes de carbone dans le groupe alcoyle et ayant 3 à 8 atomes de carbone dans le radical hétéroaryle et au moins un hétéroatome dans le radical hétéroaryle qui est choisi parmi N, O et S, les groupes alcoyle pouvant être linéaires ou ramifiés, saturés ou insaturés, aliphatiques ou alicycliques et un ou plusieurs radicaux hydrogène sur le radical hétéroaryle pouvant être remplacés par au moins un groupe qui peut être choisi indépendamment des autres parmi des groupes alcoyle ayant de 1 à 6 atomes de carbone, linéaires ou ramifiés, saturés ou insaturés, aliphatiques ou alicycliques et/ou des atomes d'halogène ; des groupes aryle et/ou arylalcoyle ayant de 1 à 6 atomes de carbone dans le radical alcoyle et ayant de 6 à 20 atomes de carbone dans le radical aryle, les groupes alcoyle pouvant être linéaires ou ramifiés, saturés ou insaturés, aliphatiques ou alicycliques et sur le radical aryle un ou plusieurs radicaux hydrogène pouvant être remplacés par au moins un groupe qui est choisi indépendamment des autres parmi des groupes alcoyle ayant de 1 à 6 atomes de carbone, linéaires ou ramifiés, saturés ou insaturés, aliphatiques ou alicycliques, et/ou des atomes d'halogène ; et des groupes -[-(CH₂)ₓ-O-]_{y}-CH₃ avec x=2à5 et y=là20; et
- le radical R est choisi parmi des groupes alcoyle ayant de 1 à 20 atomes de carbone, linéaires ou ramifiés, saturés ou insaturés, aliphatiques ou alicycliques, jusqu'à 6 radicaux hydrogènes pouvant être remplacés par des groupes OH ; des groupes hétéroaryalcoyle ayant de 1 à 6 atomes de carbone dans le radical alcoyle et ayant de 3 à 8 atomes de carbone dans le radical hétéroaryle et au moins un hétéroatome dans le radical hétéroaryle, qui est choisi parmi N, O et S, les groupes alcoyle pouvant être linéaires ou ramifiés, saturés ou insaturés, aliphatiques ou alicycliques et un ou plusieurs radicaux hydrogène du radical hétéroaryle pouvant être remplacés par au moins un groupe, qui est choisi indépendamment des autres parmi des groupes alcoyle ayant de 1 à 6 atomes de carbone linéaires ou ramifiés, saturés ou insaturés, aliphatiques ou alicycliques et/ou des atomes d'halogène ; des groupes arylalcoyle ayant de 1 à 6 atomes de carbone dans le groupe alcoyle et ayant de 6 à 20 atomes de carbone dans le radical aryle, les groupes alcoyle pouvant être linéaires ou ramifiés, saturés ou insaturés, aliphatiques ou alicycliques et un ou plusieurs radicaux hydrogène du radical aryle pouvant être substitué par au moins un groupe, qui peut être choisi indépendamment les uns des autres parmi des groupes alcoyle ayant de 1 à 6 atomes de carbone, linéaires ou ramifiés, saturés ou insaturés, aliphatiques ou alicycliques et/ou des atomes d'halogène ; et des groupes -[-(CH₂)ₓ-O-]_{y}-CH₃ avec x=2à5 et y=1à20 ;
et
II) Un constituant B), qui est constitué au moins des constituants suivants :
B3) un composé zwitterionique, qui est formé de l'un des cations mentionnés dans A), dans lesquels l'un des radicaux R, R₁, R₂ ou R₃ est un groupe -(CH₂)ₓ-SO₃ avec x = 1 à 10.

2. Constituant B) suivant la revendication 1, consistant, en outre, en au moins l'un des constituants suivants :
B1) un sel, qui est formé de l'un des anions [PO₄]³⁻, [HPO₄]²⁻, [H₂PO₄]⁻, [SO₄]²⁻, [HSO₄]⁻, [NO₃]⁻, [NO₂]⁻ ou C1⁻ et d'un, deux ou trois des cations mentionnés dans A), le nombre des cations correspondant à la valeur absolue du nombre de charge de l'anion respectif ;
B2) un sel, qui est formé d'un ou plusieurs anions bis trifluorométhylsulfonylimide 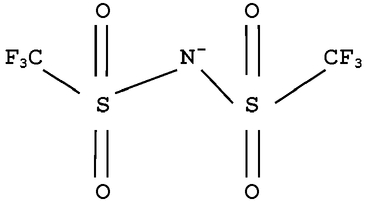 et d'un cation lithium, potassium, césium, magnésium, calcium, baryum, nickel, cobalt, fer, scandium, lanthane, zinc, gallium, cérium ou aluminium, le nombre des anions bis trifluorométhylsulfonylimide correspondant à la valeur absolue du nombre de charge du cation métallique respectif.

3. Mélange suivant la revendication 1 ou la revendication 2, **caractérisé en ce que** la proportion en masse du constituant B) dans le mélange va de 1% à 99%, en étant de préférence dans la plage de 1% à 80%.

4. Mélange suivant l'une des revendications précédentes, **caractérisé en ce que** celui-ci est liquide à une température à partir de 79°C, de préférence à partir de 49°C, d'une manière particulièrement préférée à partir de 19°C.

5. Mélange suivant l'une des revendications précédentes, **caractérisé en ce qu'**il est constitué de tributylméthylphosphonium -bis- (trifluorométhylsulfonyl) imide, de bis trifluorométhylsulfonylimide de lithium et de tributyl-4-sulfonyl-1-butanephosphonium.

6. Mélange suivant la revendication 5, **caractérisé en ce que** le rapport en masse du premier constituant mentionné au deuxième constituant mentionné au troisième constituant mentionné est dans la plage de 4 à 1 à 1, en passant par 4 à 3 à 1, jusqu'à 4 à 3 à 3.

7. Procédé pour effectuer une synthèse du méthanol en utilisant du monoxyde de carbone et de l'hydrogène, du dioxyde de carbone et de l'hydrogène ou un mélange de monoxyde de carbone, de dioxyde de carbone et d'hydrogène comme éduits de synthèse dans un réacteur à une température dans la plage de 100°C et 300°C et une pression de gaz de synthèse dans la plage de 50 bar à 300 bar en présence d'un catalyseur comprenant les stades suivants :
- on met un mélange liquide suivant l'une des revendications 1 à 6 dans le réacteur ;
- on fait réagir du monoxyde de carbone et de l'hydrogène, du dioxyde de carbone et de l'hydrogène ou un mélange de monoxyde de carbone, de dioxyde de carbone et d'hydrogène en le produit de synthèse méthanol ou en les produits de synthèse méthanol et eau dans le réacteur à une température dans la plage de 100°C et 300°C et à une pression de gaz de synthèse dans la plage de 50 bar à 300 bar ;
- on sorbe au moins une quantité partielle d'au moins un produit de synthèse de la phase gazeuse, contenant le au moins un produit de synthèse, dans le mélange liquide ; et
- on fait sortir en continu ou en discontinu le mélange liquide du réacteur.

8. Procédé suivant la revendication 7, **caractérisé en ce que** l'on introduit la phase gazeuse contenant au moins un produit de synthèse dans le mélange au moyen d'un agitateur d'introduction de gaz.

9. Procédé suivant la revendication 7 ou 8, **caractérisé en ce que** l'on détend le mélange liquide sorti du réacteur et **en ce que** l'on recueille le méthanol, séparé ainsi au moins en partie du mélange.

10. Procédé suivant la revendication 9, **caractérisé en ce qu'**après la détente du mélange liquide et avoir séparé du mélange au moins partiellement le produit de synthèse méthanol ou des produits de synthèse méthanol et eau, on retourne le mélange au réacteur.

11. Procédé suivant l'une des revendications 7 à 10, **caractérisé en ce qu'**on utilise comme catalyseur de l'oxyde de cuivre et de zinc sur de l'oxyde d'aluminium, qui est mis dans la phase gazeuse du gaz de synthèse du réacteur.

12. Procédé suivant l'une des revendications 7 à 11, **caractérisé en ce que** l'on utilise comme gaz de synthèse du dioxyde de carbone et de l'hydrogène, on absorbe dans le réacteur dans le mélange liquide au moins des quantités partielles des produits de synthèse méthanol et eau, après avoir fait sortir le mélange du réacteur et avoir détendu le mélange, on sépare les produits de synthèse méthanol et eau au moins en partie du mélange, on recueille au moins le produit de synthèse méthanol séparé au moins en partie et, après détente du mélange liquide et avoir séparé du mélange au moins partiellement les produits de synthèse, on retourne le mélange au réacteur.

13. Procédé suivant l'une des revendications 10 à 12, **caractérisé en ce qu'**on retire de la chaleur du mélange avant de le renvoyer au réacteur.
